Europäisches Patentamt

European Patent Office

Office européen des brevets

(19)

(11) Numéro de publication : **0 261 191 B1**

(12) **FASCICULE DE BREVET EUROPEEN**

(45) Date de publication du fascicule du brevet :
**27.11.91 Bulletin 91/48**

(51) Int. Cl.⁵ : **C07C 227/28**

(21) Numéro de dépôt : **87902107.9**

(22) Date de dépôt : **26.03.87**

(86) Numéro de dépôt international :
**PCT/FR87/00094**

(87) Numéro de publication internationale :
**WO 87/05895 08.10.87 Gazette 87/22**

(54) **PROCEDE DE PREPARATION INDUSTRIELLE D'ACIDES AMINES PAR HYDROLYSE DE PROTEINES EN MILIEU ACIDE.**

(30) Priorité : **27.03.86 FR 8604441**

(43) Date de publication de la demande :
**30.03.88 Bulletin 88/13**

(45) Mention de la délivrance du brevet :
**27.11.91 Bulletin 91/48**

(84) Etats contractants désignés :
**AT BE CH DE FR GB IT LI LU NL SE**

(56) Documents cités :
**US-A- 2 457 117**
**US-A- 2 657 232**
**US-A- 3 048 627**

(73) Titulaire : **LABORATOIRES FLORK S.A.**
**Zone Industrielle du Brézet 10, rue Thimonier**
**F-63100 Clermont-Ferrand (FR)**

(72) Inventeur : **FLORK, Michel**
**60, rue de Bellevue**
**F-63400 Chamalières (FR)**

(74) Mandataire : **Rinuy, Santarelli**
**14, avenue de la Grande Armée**
**F-75017 Paris (FR)**

# Description

La présente invention a pour objet un nouveau procédé industriel d'hydrolyse de protéines pour l'extraction d'acides amines.

Il est deja connu d'extraire les acides amines contenus dans des protéines par hydrolyse de ces dernières avec des acides minéraux forts.

Pour assurer la rupture des liaisons peptidiques des protéines et la formation des acides aminés correspondants avec un bon rendement, il est nécessaire que cet acide ait un pKA inférieur ou égal à - 2.

Mais cet acide fort ne doit pas provoquer la dégradation des acides aminés formés au cours de la réaction d'hydrolyse et dont certains sont instables en milieu acide concentré et l'excès de cet acide doit être facile à éliminer du mélange réactionnel après hydrolyse.

Des essais concluants à cet égard ont été obtenus en laboratoire avec des solutions aqueuses d'acide chlorydrique, dont le pKA est de -7, à une concentration d'environ 6N et à une température égale ou supérieure à 100°C.

Mais l'extension de ces essais à l'échelle industrielle pose des problèmes difficiles car l'acide chlorhydrique s'il est facile à éliminer par distillation, donne lieu aux températures auxquelles a lieu l'hydrolyse, à des émissions de vapeurs acides qui nécessitent d'opérer en vase clos et d'effectuer l'hydrolyse des protéines par lots complets.

Or la faible densité apparente de certaines sources de ces protéines, comme les soies de porc qui ont une densité apparente de 0,2, ne permet pas de charger dans le réacteur d'hydrolyse un volume important de ces protéines sans un accroissement démesuré du volume de ce réacteur.

De plus l'acide chlorhydrique est extrêmement corrosif et nécessite l'emploi de réacteurs vitrifiés particulièrement coûteux.

L'emploi de l'acide sulfurique au lieu de l'acide chlorhydrique pour l'hydrolyse des protéines a également été proposé notamment dans les brevets US-A-2.048.627, US-A-2.457.117 et US-A-2.657.232.

Toutefois dans tous ces brevets, la totalité de l'acide sulfurique, concentré ou dilué, était utilisé dès le départ de la réaction d'hydrolyse des protéines, pendant plusieurs heures à une température de plus de 100°C ; il en résultait une concentration en acide sulfurique, qui variant pendant toute la durée de la réaction d'hydrolyse, se traduisait par un mauvais rendement en acides aminés et des résultats non reproductibles.

De plus, l'emploi de l'acide sulfurique concentré ne permet d'obtenir ni un bon rendement en acides aminés ni une hydrolyse convenable par suite de la dégradation de certains d'entre eux.

Le procédé selon la présente invention permet de surmonter ces inconvénients en faisant appel à des conditions particulières d'hydrolyse par l'acide sulfurique, dont seule la première acidité de pKA égale à -7,5 est suffisamment forte pour intervenir dans l'ouverture des liaisons peptidiques.

L'emploi de l'acide sulfurique pour l'hydrolyse des protéines est surprenant car sa seconde acidité n'intervient pas dans la réaction d'hydrolyse et risquait à priori de nuire à la stabilité des acides aminés en cours de formation.

Il en est d'autant plus ainsi que cet acide est à utiliser dans le cadre du procédé selon la présente invention à une concentration qui doit être maintenue tout au long de la réaction d'hydrolyse à une teneur minimale de 12N pour que la dissolution des protéines puisse s'effectuer et que l'hydrolyse ait lieu avec un bon rendement.

Il convient de noter que cette concentration minimale de ce diacide est le double de celle qui était utilisée pour l'acide chlorhydrique.

L'utilisation de ces conditions d'hydrolyse à l'acide sulfurique selon la présente invention a des conséquences importantes tant sur le plan économique que sur le plan pratique.

Tout d'abord, l'hydrolyse en milieu sulfurique peut se faire dans des cuves comportant un revêtement de protection simple, solution beaucoup moins onéreuse que les revêtements vitrifiés indispensables si l'hydrolyse avait lieu en milieu chlorhydrique.

Ensuite, la température d'ébullition d'un mélange sulfurique contenant les protéines à hydrolyser en cours de dissolution n'intervenant qu'à 120°C, l'hydrolyse peut être conduite facilement à la température minimale de 100°C nécessaire pour obtenir un bon rendement, sans émission de vapeurs acides, ce qui n'est pas le cas de l'acide chlorhydrique.

Enfin et surtout, étant donné qu'on peut effectuer la réaction d'hydrolyse sulfurique à l'air libre, il va être possible d'introduire progressivement dans la cuve le volume des protéines à hydrolyser et d'introduire également progressivement la quantité d'acide sulfurique qui va être consommée au cours de la réaction d'hydrolyse.

Une telle manière d'opérer, par addition progressive des protéines et de l'acide dans le milieu réactionnel, soit en continu soit par apports successifs, permet de maintenir dans une fourchette étroite la concentration en acide sulfurique pendant toute la durée de l'hydrolyse, ce qui améliore le rendement en acides aminés.

On obtient en effet, dans ces conditions, une dissociation suffisante des liaisons peptidiques tout en contrôlant la réaction d'hydrolyse pour éviter toute dégradation des acides aminés produits.

Le procédé faisant l'objet de la présente invention pour la préparation industrielle d'acides aminés par hydrolyse de protéines d'origine animale ou végétale consiste à effectuer l'hydrolyse de ces protéines par chauffage dans une cuve en présence d'une solution

ce qui fait monter la temperature à 105-110°C.

On verse alors dans la cuve 15 tonnes de soie de porc à la cadence d'environ 2,250 tonnes à l'heure en coulant simultanément 1 mètre cube à l'heure supplémentaire d'acide sulfurique à 92 %.

Après avoir maintenu la température entre 105 et 110°C et avoir continue l'agitation pendant 4 heures après la fin de l'introduction des 15 tonnes de soies et des 6,67 mètres cubes d'acide on arrête l'hydrolyse en ajoutant dans la cuve 20 à 25 m³ d'eau, tout en poursuivant l'agitation.

On effectue ensuite la neutralisation en coulant dans un réacteur des fractions de 5 m³ chacune, du jus d'hydrolyse, auxquelles on ajoute 2 m³ d'eau et qu'on saupoudre lentement avec 1300 kg de chaux éteinte.

On agite pendant 2 heures chacune de ces fractions du jus d'hydrolyse en évitant la formation de mousse, en maintenant la température à moins de 50°C et en vérifiant que le pH atteint une valeur comprise entre 1,5 et 2 et enfin on coule dans un bassin de décantation.

L'opération de neutralisation est répétée 10 fois pour vider complètement la cuve d'hydrolyse ce qui correspond à une consommation de 13.000 kg de chaux éteinte pour environ 50 m³ de jus d'hydrolyse.

Après coulée dans le bassin de décantation, une mousse plus ou moins importante et stable peut surnager.

Il convient alors d'arroser la surface du bassin avec un jet d'eau pulvérisée pour briser la mousse.

On laisse décanter une nuit entière et on récupère le jus d'hydrolyse limpide qui contient en moyenne, après hydrolyse de 13 tonnes de soie de porc, 12500 kilogs d'acides aminés totaux, dont en particulier :

| 750 | kilogs | d'acide aspartique |
|---|---|---|
| 650 | " | de thréonine |
| 1350 | " | de sérine |
| 1900 | " | d'acide glutamique |
| 900 | " | de proline |
| 650 | " | de glycine |
| 600 | " | d'alanine |
| 800 | " | de valine |
| 1150 | " | de cystine |
| 100 | " | de méthionine |
| 550 | " | d'isoleucine |
| 1100 | " | de leucine |
| 400 | " | de tyrosine |
| 450 | " | de phénylalanine |
| 650 | " | de lysine |
| 250 | " | d'histidine |
| et 1200 | " | d'arginine |

L'exemple ci-dessus et les considérations qui précèdent ne sauraient limiter la portée de la présente invention qui concerne tout procédé industriel d'hydrolyse des protéines pour l'extraction des acides aminés mettant en oeuvre une ou plusieurs des revendications suivantes.

## Revendications

1. Procédé de préparation industrielle d'acides aminés par hydrolyse de protéines d'origine animale ou végétale consistant à effetuer l'hydrolyse des protéines par chauffage dans une cuve en présence d'une solution d'acide sulfurique au moins égale à 12N à une température d'au moins 100°C, à arrêter l'hydrolyse par addition d'eau pour abaisser la température et réduire la concentration d'acide sulfurique à une valeur au plus égale à 6N et à éliminer l'excédent d'acide sulfurique, caractérisé par le chargement préalable de la cuve réactionnelle avec une partie de la solution d'acide sulfurique nécessaire à l'hydrolyse des protéines et son chauffage à une température maintenue à au moins 100°C, et par l'addition simultanée d'une part, des protéines à hydrolyser et, d'autre part, de la quantité complémentaire de la solution d'acide sulfurique correspondant à celle nécessaire à la neutralisation des fonctions amines des acides aminés au fur et à mesure de leur formation par hydrolyse des protéines de façon à maintenir la concentration en acide sulfurique sensiblement au

d'acide sulfurique au moins égale à 12N à une température d'au moins 100°C, à arrêter l'hydrolyse par addition d'eau pour abaisser la température et réduire la concentration d'acide sulfurique à une valeur au plus égale à 6N et à éliminer l'excédent d'acide sulfurique, et il se caractérise par le chargement préalable de la cuve réactionnelle avec une partie de la solution d'acide sulfurique nécessaire à l'hydrolyse des protéines et son chauffage à une température maintenue à au moins 100°C et par l'addition simultanée d'une part, des protéines à hydrolyser et, d'autre part, de la quantité complémentaire d'acide sulfurique correspondant à celle nécessaire à la neutralisation des fonctions amines des acides aminés au fur et à mesure de leur formation par hydrolyse des protéines de façon à maintenir la concentration en acide sulfurique sensiblement au niveau de départ.

Grâce à l'emploi de l'acide sulfurique, il n'est plus indispensable de travailler en vase clos et d'introduire dans la cuve dès le début de la réaction la totalité des matières premières nécessaires à l'hydrolyse des protéines.

Au contraire, on n'introduit au départ dans la cuve d'hydrolyse qu'une partie, de l'ordre de 40 à 60 % en poids, de la totalité de l'acide sulfurique utilisée dans la réaction.

Et, après mise à la température voulue, on introduit ensuite dans la cuve les protéines à hydrolyser et la quantité complémentaire d'acide sulfurique correspondant à la neutralisation des fonctions amines, de préférence de manière simultanée, les débits étant ajustés d'une part par réglage des volumes absolus de protéines et d'acide sulfurique complémentaire ajoutés dans la cuve pour assurer une température aussi constante que possible et d'autre part par réglage des volumes relatifs de ces deux ingrédients ainsi ajoutés pour maintenir la concentration en acide sulfurique sensiblement au niveau de départ.

Cette introduction différée dans le temps des matières premières nécessaires à l'hydrolyse, qui n'aurait pas été possible en milieu chlorhydrique, constitue une caractéristique importante du procédé selon l'invention.

Elle permet tout d'abord de maintenir beaucoup plus constante l'acidité tout au long de la réaction d'hydrolyse, la concentration en acide pouvant être maintenue entre 12N et 14N pour éviter toute dégradation des acides aminés, alors qu'avec un chargement initial de toute la matière première il faudrait partir d'un mélange dont la concentration en acide serait de 20N à 25N.

Elle permet également d'introduire dans une cuve de volume donné un volume beaucoup plus important de protéines à hydrolyser puisque ces dernières sont dissoutes au fur et à mesure de leur introduction.

Elle permet enfin d'éviter la transformation des acides aminés en d'autres acides aminés ou leur racémisation avec obtention de la forme dextrogyre au lieu de la forme lévogyre.

Il y a donc simultanément dissolution des protéines, hydrolyse de leurs liaisons peptidiques et neutralisation des acides aminés, ces différentes réactions s'effectuant avec leur propre cinétique, qui diffère selon chacun des acides aminés concernés.

Les protéines utilisées comme matières premières pour le procédé selon l'invention peuvent être d'origine animale ou végétale comme la kératine des cheveux, les poils ou les plumes des animaux, la soie de porc en provenance des abattoirs étant la source de protéine préférée.

Pour obtenir un bon rendement, même avec des quantités importantes de protéines, on maintient le mélange réactionnel sous agitation à une température de préférence de 105°C pendent une durée suffisante pour permettre le dissolution et l'hydrolyse des protéines dans le bain d'acide sulfurique.

Lorsque l'hydrolyse est pratiquement terminée, on arrête le processus d'hydrolyse par adjonction d'eau pour abaisser le température vers 60°C environ et réduire le concentration en acide sulfurique de 12N à une valeur eu plus égale à 6N. On évite ainsi des réactions secondaires entraînant le dégradation des acides aminés et des pertes de rendement.

Le dernière étape du procédé consiste à éliminer les ions sulfuriques excédentaires et les diverses substances orgqniques, colorants et corps gras afin d'obtenir un hydrolysat convenent pour l'isolement et le purification des acides aminés dissous qu'il contient.

Dans un mode préféré de mise en oeuvre de l'invention, on précipite les ions sulfuriques sous forme de sulfate de calcium par addition de chaux éteinte pour limiter l'exothermie et éviter des dégagements de gaz carbonique. Elle a lieu de préférence à un pH compris entre 1 et 2 dans un réacteur à double enveloppe refroidi. Cette précipitation s'effectue sous agitation en un temps relativement court de l'ordre de 2 à 4 heures pour éviter que les grains de chaux ne soient totalement transformés en sulfate de calcium, la cristallisation du sulfate de calcium sur des grains de chaux permettent d'augmenter la granulométrie du précipité et de faciliter sa séparation ultérieure. Au cours de cette précipitation, les acides gras sont entraînés par adsorption sur les sulfates.

La séparation du précipité de sulfate de calcium s'effectue par décantation ou filtration.

L'invention va maintenant être illustrée à l'aide d'un exemple de réalisation industrielle.

On charge dans une cuve de 50m³ en acier ordinaire revêtue intérieurement d'hypalon (marque de fabrique), 8,5 mètres cubes d'eau.

On chauffe à la vapeur vive jusqu'à une température comprise entre 90°C et 100°C, ce qui amène une quantité supplémentaire d'eau de l'ordre de 2,1 m³ et on verse rapidement sous agitation 5,7 m³ d'acide sulfurique à 92 % ayant une densité de 1,83,

niveau de départ.

2. Procédé selon la revendication 1 caractérisé en ce que les protéines d'origine animale sont de la soie de porc.

3. Procédé selon l'une quelconque des revendications 1 ou 2 caractérisé en ce que la proportion d'acide chargée au préalable dans la cuve avant l'introduction des protéines est de 40 à 60 °% en poids par rapport à la quantité totale d'acide sulfurique utilisée dans la réaction d'hydrolyse.

4. Procédé selon l'une quelconque des revendications précédentes caractérisé en ce qu'on élimine l'excédent d'acide sulfurique après l'hydrolyse par addition de chaux éteinte et décantation du précipité de sulfate de calcium.

5. Procédé selon la revendication 4 caractérisé en ce que l'addition de chaux éteinte s'effectue sous agitation à une température ne dépassant pas 50°C jusqu'à obtention d'un pH compris entre 1 et 2.


**Patentansprüche**

1. Verfahren zur industriellen Herstellung von Aminosäuren durch Hydrolyse von Proteinen tierischen oder pflanzlichen Ursprungs, das darin besteht, daß die Hydrolyse von Proteinen durch Erhitzen in einem Behälter in Anwesenheit einer mindestens 12 n Schwefelsäurelösung auf eine Temperatur von mindestens 100°C durchgeführt wird, die Hydrolyse durch Zusetzen von Wasser zum Senken der Temperatur und Vermindern der Schwefelsäurekonzentration auf einen Wert von höchstens 6 n abgebrochen wird und der Schwefelsäureüberschuß entfernt wird, gekennzeichnet durch vorherige Beladung des Reaktionsbehälters mit einem Teil der Schwefelsäurelösung, die zur Hydrolyse der Proteine erforderlich ist, und Erhitzen auf eine Temperatur von mindestens 100°C und durch gleichzeitiges Zusetzen einerseits der zu hydrolysierenden Proteine und andererseits der restlichen Menge der Schwefelsäurelösung entsprechend jener, die zur Neutralisation der Aminfunktionen der Aminosäuren notwendig ist, im Ausmaß ihrer Bildung durch Hydrolyse der Proteine, so daß die Konzentration der Schwefelsäure etwa auf Ausgangshöhe gehalten wird.

2. Verfahren nach Anspruch 1, dadurch gekennzeichnet, daß die Proteine tierischen Ursprungs von Schweineborsten stammen.

3. Verfahren nach einem der Ansprüche 1 oder 2, dadurch gekennzeichnet, daß der Säureanteil, der vorher in den Behälter eingebracht wird, vor Einbringen der Proteine 40 bis 60 Gew.%, bezogen auf die gesamte bei der Hydrolysereaktion verwendete Schwefelsäuremenge, beträgt.

4. Verfahren nach einem der vorhergehenden Ansprüche, dadurch gekennzeichnet, daß der Überschuß der Schwefelsäure nach der Hydrolyse durch Zusatz von Löschkalk und Dekantieren des Calciumsulfatniederschlages entfernt wird.

5. Verfahren nach Anspruch 4, dadurch gekennzeichnet, daß der Zusatz von Löschkalk unter Rühren bei einer 50°C nicht übersteigenden Temperatur durchgeführt wird, bis ein pH zwischen 1 und 2 erreicht ist.


**Claims**

1. Process for the industrial preparation of amino acids by hydrolysis of proteins of animal or vegetable origin, which comprises effecting the hydrolysis of the proteins by heating in a tank in the presence of a sulphuric acid solution of at least 12 N strength to a temperature of at least 100°C, stopping the hydrolysis by adding water to lower the temperature and reduce the sulphuric acid concentration to a value of at most 6 N, and removing the excess sulphuric acid, characterised in that the reaction tank is charged beforehand with a part of the sulphuric acid solution required for the hydrolysis of the proteins and this solution is heated to a temperature kept at at least 100°C, and in that there are simultaneously added, on the one hand, the proteins to be hydrolysed and, on the other hand, the complementary amount of the sulphuric acid solution corresponding to that required to neutralise the amine groups of the amino acids at the rate at which they are formed by hydrolysis of the proteins, so as to keep the concentration of sulphuric acid substantially at its starting level.

2. Process according to Claim 1, characterised in that the proteins of animal origin are pig's bristles.

3. Process according to either of Claims 1 or 2, characterised in that the proportion of acid introduced initially into the tank before the introduction of the proteins is from 40 to 60% by weight relative to the total amount of sulphuric acid used in the hydrolysis reaction.

4. Process according to any of the preceding claims, characterised in that the excess sulphuric acid is removed, after hydrolysis, by adding slaked lime and decanting the calcium sulphate precipitate.

5. Process according to Claim 4, characterised in that the addition of slaked lime is carried out with stirring at a temperature not exceeding 50°C until a pH of between 1 and 2 is reached.